# EUROPÄISCHE PATENTSCHRIFT

(11) **EP 0 896 537 B1**
(45) Veröffentlichungstag und Bekanntmachung des Hinweises auf die Patenterteilung: **18.08.1999**
(21) Anmeldenummer: 97907081.0
(22) Anmeldetag: 07.03.1997
(51) Int. Cl.: A61K 31/42, A61K 31/275

(54) **KOMBINATIONSPRÄPARAT, ENTHALTEND 5-METHYLISOXAZOL-4-CARBONSÄURE-(4-TRIFLUORMETHYL)-ANILID UND N-(4-TRIFLUORMETHYLPHENYL)-2-CYAN-3-HYDROXYCROTONSÄUREAMID**
PREPARATION CONTAINING A COMBINATION OF 5-METHYLISOXAZOLE-4-CARBOXYLIC ACID-(4-TRIFLUOROMETHYL)-ANILIDE AND N-(4-TRIFLUOROMETHYLPHENYL)2-CYANO-3-HYDROXYCROTONIC ACID AMIDE
PREPARATION COMPRENANT UNE COMBINAISON DE 5-METHYLISOXAZOL-4-ACIDE CARBOXYLIQUE-(4-TRIFLUOROMETHYL)-ANILIDE ET DE N-(4-TRIFLUOROMETHYLPHENYL)-2-CYANO-3-ACIDE HYDROXYCROTONIQUE AMIDE

(30) Priorität: 20.03.1996 DE 19610955
(43) Veröffentlichungstag der Anmeldung: 17.02.1999
(73) Patentinhaber: HOECHST AKTIENGESELLSCHAFT, 65929 Frankfurt am Main (DE)
(72) Erfinder: BARTLETT, Robert, D-64291 Darmstadt (DE); THEN, Johann, D-65931 Frankfurt (DE)
(86) Internationale Anmeldenummer: EP9701167
(87) Internationale Veröffentlichungsnummer: WO9734600

(56) Entgegenhaltungen:
- EP-A- 0 217 206
- EP-A- 0 665 013
- WO-A-91/17748
- DE-A- 4 127 737

## Beschreibung

Aus der Europäischen Patentanmeldung, Veröffentlichungsnummer 0 013 376, ist bekannt, daß 5-Methylisoxazol-4-carbonsäure-(4-trifluormethyl)-anilid (Verbindung 1) antirheumatisch, antiphlogistisch, antipyretisch und analgetisch wirksam ist und gegen multiple Sklerose eingesetzt werden kann. Arzneimittel, enthaltend den Wirkstoff 5-Methylisoxazol-4-carbonsäure-(4-trifluormethyl)-anilid werden in Dosen von 25 mg bis 150 mg oral appliziert.

In der Europäischen Patentanmeldung, Veröffentlichungsnummer 0 217 206, wird beschrieben, daß N-(4-Trifluormethylphenyl)-2-cyan-3-hydroxycrotonsäureamid (Verbindung 2) immunmodulierende Eigenschaften hat und sich zur Behandlung von chronischer Graft-versus-Host-Krankheit und Autoimmunerkrankungen, insbesondere systemischem Lupus erythematodes, eignet. Pharmazeutische Präparate, enthaltend eine Verbindung 1 oder 2 in einer Dosis von 10 bis 200 mg, bevorzugt jedoch 50 bis 100 mg, bei Injektionslösung in Ampullenform (intravenös), insbesondere auf Basis der Verbindung 2 oder eines Salzes davon, 1 bis 30 mg, vorzugsweise 5 bis 10 mg und bei rektaler Verabreichung 50 bis 300 mg, vorzugsweise 100 bis 200 mg können appliziert werden. Jedoch zeigt die orale Applikation von 5 mg/kg oder 10 mg/kg von der Verbindung 1 oder 2 jeweils alleine keine signifikante Wirkung.

Es wurde gefunden, daß ein Kombinationspräparat, enthaltend die Verbindungen 1 und 2, überraschend günstige immunsuppressive Effekte aufweist. Der Zusatz geringer Mengen von Verbindung 2 zur Hauptwirkkomponente Verbindung 1, führt zu einer deutlichen Wirkungssteigerung des Kombinationspräparats. Aufgrund des Ausmaßes dieses Effekts läßt sich die Anwendung dieser Kombination auf Bereiche ausdehnen, die einer immunsuppressiven Therapie durch die Einzelkomponenten bislang verschlossen war. Ferner führt die Dosisreduzierung ohne verringerte Wirksamkeit zur erhöhten Sicherheit bei der Anwendung. Gleichzeitig ist davon auszugehen, daß die Therapiekosten über eine Dosiserniedrigung bei gleichbleibender Wirksamkeit in nennenswertem Umfang gesenkt werden können.

Die Erfindung betrifft daher eine feste Zubereitung, enthaltend die Komponente 1) 5-Methylisoxazol-4-carbonsäure-(4-trifluormethyl)-anilid, die Komponente 2) N-(4-Trifluormethylphenyl)-2-cyan-3-hydroxycrotonsäureamid und/oder ein physiologisch verträgliches Salz von N-(4-Trifluormethylphenyl)-2-cyan-3-hydroxycrotonsäureamid und/oder eine stereoisomere Form von N-(4-Trifluormethylphenyl)-2-cyan-3-hydroxycroton-säureamid und 3) einen pharmazeutischen Träger,
die dadurch gekennzeichnet ist, daß der Gehalt der Komponente 1 von 2 bis 20 mg beträgt und der Gehalt der Komponente 2) von 0,3 % bis 50 % der Komponente 1) beträgt.

Zusammensetzungen enthaltend die Verbindungen 1 oder 2 sind bekannt aus EP-A-665013 und DE-A-4 127 737.

Die Verbindungen 5-Methylisoxazol-4-carbonsäure-(4-trifluormethyl)-anilid und N-(4-Trifluormethylphenyl)-2-cyan-3-hydroxy-crotonsäureamid lassen sich nach bekannten Verfahren herstellen (EP 0 529 500). In der Zubereitung gemäß der vorliegenden Erfindung wird N-(4-Trifluormethylphenyl)-2-cyan-3-hydroxycrotonsäureamid mit folgender Strukturformel 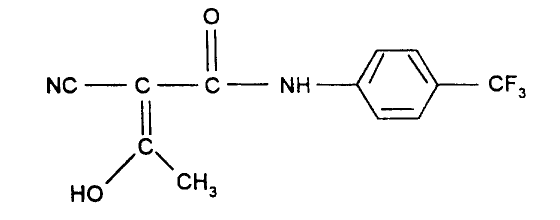 als solches eingesetzt und/oder ein physiologisch verträgliches Salz von N-(4-Trifluormethylphenyl)-2-cyan-3-hydroxycrotonsäureamid und/oder eine stereoisomere Form von N-(4-Trifluormethylphenyl)-2-cyan-3-hydroxycrotonsäureamid.

Geeignete physiologisch verträgliche Salze von N-(4-Trifluormethylphenyl)-2-cyan-3-hydroxy-crotonsäureamid sind beispielsweise Alkali-, Erdalkali- oder Ammoniumsalze, einschließlich solcher von physiologisch verträglichen organischen Ammoniumbasen.

Die erfindungsgemäße feste Zubereitung eignet sich beispielsweise zur Behandlung von
- akuten immunologischen Ereignissen wie Sepsis, Allergie, Graft-versus-Host- und Host-versus-Graft-Reaktionen
- Autoimmunerkrankungen, insbesondere rheumatoide Arthritis, systemischem Lupus erythematodes, multipler Sklerose
- Psoriasis, atopischer Dermatitis, Asthma, Urtikaria, Rhinitis, Uveitis
- Typ II-Diabetes
- Leberfibrose, zystischer Fibrose, Kolitis
- Krebserkrankungen wie Lungenkrebs, Leukämie, Eierstockkrebs, Sarkome, Kaposi's Sarkom, Meningiom, Darmkrebs, Lymphknotenkrebs, Hirntumore, Brustkrebs, Pankreaskrebs, Prostatakrebs oder Hautkrebs.

Die erfindungsgemäße feste Zubereitung kann auch Kombinationspackungen oder Kompositionen umfassen, in denen die Bestandteile nebeneinandergestellt sind und deshalb gleichzeitig, getrennt oder zeitlich abgestuft an ein und demselben menschlichen oder tierischen Körper angewendet werden können. Erfindungsgemäß können die Komponenten 1 und 2 auch in nebeneinanderliegenden, getrennten Arzneiformen vorliegen, insbesondere dann, wenn die Arzneiformen von den räumlichen Abmessungen her eine Applikation erschweren. Dies gilt besonders für die oralen Formen, da häufig bei älteren Patienten eine Abneigung gegen große Tabletten oder Kapseln vorherrscht. Zwingend ist, daß die getrennt, nebeneinander vorliegenden Arzneiformen zur zeitlich gemeinsamen Einnahme hergerichtet sind. Dabei können auch unterschiedliche Formen, z.B. Tablette und Kapsel, nebeneinander vorliegen.

Die Erfindung betrifft ferner die Verwendung einer Kombination der Verbindungen 1 und 2 zur Herstellung eines Arzneimittels mit überadditiver Steigerung der immunsuppressiven Wirkung.

Die Erfindung betrifft ferner ein Verfahren zur Herstellung der erfindungsgemäßen Zubereitung, das dadurch gekennzeichnet ist, daß man die Verbindungen 1 und 2 und einen pharmazeutischen Träger zu einer pharmazeutischen Darreichungsform verarbeitet.

Die erfindungsgemäße feste Zubereitung kann als Dosiereinheit in Form von Arzneiformen wie Kapseln (einschließlich Mikrokapseln), Tabletten (einschließlich Dragees und Pillen) oder Zäpfchen vorliegen, wobei bei Verwendung von Kapseln das Kapselmaterial die Funktion des Trägers wahrnehmen und der Inhalt z.B. als Pulver, Gel, Emulsion, Dispersion oder Lösung vorliegen kann. Besonders vorteilhaft und einfach ist es jedoch, orale (perorale) Formulierungen mit den beiden Verbindungen 1 und 2 herzustellen, die die berechneten Mengen der Wirkstoffe zusammen mit jedem gewünschten pharmazeutischen Träger enthalten. Auch eine entsprechende Formulierung (Zäpfchen) für die rektale Therapie kann angewandt werden. Ebenso ist die transdermale Applikation in Form von Salben, Cremes oder orale Applikation von Lösungen, die die erfindungsgemäße Zubereitung enthalten, möglich.

Salben, Pasten, Cremes und Puder können neben den Wirkstoffen die üblichen Trägerstoffe enthalten, z.B. tierische und pflanzliche Fette, Wachse, Paraffine, Stärke, Tragacanth, Zellulosederivate, Polyethylenglykole, Silicone, Bentonite, Talkum, Zinkoxid, Milchzucker, Kieselsäure, Aluminiumhydroxid, Kalziumsilikat und Polyamidpulver oder Gemische dieser Stoffe.

Die Tabletten, Pillen oder Granulatkörper können nach üblichen Verfahren wie Preß-, Tauch- oder Wirbelbettverfahren oder Kesseldragierung hergestellt werden und enthalten Trägermittel und andere übliche Hilfsstoffe wie Gelatine, Agarose, Stärke (z.B. Kartoffel-, Mais- oder Weizenstärke), Cellulose wie Ethylcellulose, Siliziumdioxid, verschiedene Zucker wie Milchzucker, Magnesiumcarbonat und/oder Kalziumphosphate. Die Dragierlösung besteht gewöhnlich aus Zucker und/oder Stärkesirup und enthält meistens noch Gelatine, Gummi arabicum, Polyvinylpyrrolidon, synthetische Zelluloseester, oberflächenaktive Substanzen, Weichmacher, Pigmente und ähnliche Zusätze entsprechend dem Stand der Technik. Zur Herstellung der Zubereitungen kann jedes übliche Fließregulierungs-, Schmier- oder Gleitmittel wie Magnesiumstearat und Trennmittel verwendet werden.

Bevorzugt haben die Zubereitungen die Form von Mantel-/Kern-Tabletten oder Mehrschichttabletten, wobei sich die Verbindung 2 im Mantel oder im Kern oder in einer Schicht befindet, während sich die Verbindung 1 im Kern oder im Mantel oder in einer anderen Schicht befindet. Die Verbindungen 1 und 2 können auch in retardierter Form vorliegen oder an Retardierungsmaterial adsorbiert oder im Retardierungsmaterial (z.B. solches auf Cellulose- oder Polystyrolharzbasis, z.B. Hydroxyethylcellulose) eingeschlossen sein. Eine verzögerte Freisetzung der Wirkstoffe kann auch erreicht werden, indem die betreffende Schicht oder das Kompartiment mit üblichen magensaftunlöslichen Überzügen versehen wird.

Die anzuwendende Dosierung ist selbstverständlich abhängig von verschiedenen Faktoren wie dem zu behandelnden Lebewesen (d.h. Mensch oder Tier), Alter, Gewicht, allgemeiner Gesundheitszustand, dem Schweregrad der Symptome, der zu behandelnden Erkrankung, eventuellen Begleiterkrankungen, (falls vorhanden) der Art der begleitenden Behandlung mit anderen Arzneimitteln, oder der Häufigkeit der Behandlung. Die Dosierungen werden im allgemeinen mehrfach pro Tag und vorzugsweise einmal bis dreimal pro Tag verabreicht. Die verwendeten Mengen an Einzelwirkstoff orientieren sich hierbei an der empfohlenen Tagesdosis des jeweiligen Einzelwirkstoffs und sollen im allgemeinen im Kombinationspräparat von 10 % bis 100 % der empfohlenen Tagesdosis liegen, bevorzugt von 20 % bis 80 %, insbesondere bei 50 %. Die geeignete Therapie mit den erfindungsgemäßen Kombinationen besteht somit z.B. in der Verabreichung von einer, zwei oder 3 Einzeldosierungen der Zubereitung bestehend aus
1) 5-Methylisoxazol-4-carbonsäure-(4-trifluormethyl)-anilid in einer Menge von 2 bis 20 mg, 2 bis 19,9 mg, 4,5 bis 19,5 mg, 4,85 bis 19 mg, 5 bis 18 mg, 5 bis 15 mg, 5 bis 10 mg, 5 bis 9,9 mg, 5 bis 9,7 mg oder 5 bis 9,0 mg und
2) N-(4-Trifluormethylphenyl)-2-cyan-3-hydroxy-crotonsäureamid in einer Menge von 0,3 % bis 50 %, bevorzugt von 0,5 % bis 20 %, insbesondere von 0,8 % bis 15 %, insbesondere bevorzugt von 1 % bis 10 %, ganz besonders bevorzugt von 1 % bis 5 %; jeweils bezogen auf den Gehalt von 5-Methylisoxazol-4-carbonsäure-(4-trifluormethyl)-anilid, und
3) einen pharmazeutisch verträglichen Träger.

Die Prozentangaben (%) der Verbindungen 1 und 2 beziehen sich jeweils auf Gewichtsprozente.

Die Menge der Wirkkomponenten hängt naturgemäß von der Zahl der Einzeldosierungen und auch von der zu behandelnden Krankheit ab. Die Einzeldosierung kann auch aus mehreren, gleichzeitig verabreichten Dosiereinheiten bestehen.

### Beispiel 1

### Pharmakologische Prüfungen

### Adjuvans induzierte Arthritis, Modifikation nach Perper (Proc. Soc. exp. Biol. Med. 137, 506 (1971)

Als Versuchstiere dienen männliche Ratten eines Lewis-Stammes (Moellegard, Dänemark) mit einem Körpergewicht von 160 bis 210 g. Die Tiere erhalten am 1. Tag eine subkutane Injektion in die Schwanzwurzel mit kompletten Freund'schen Adjuvans enthaltend eine Mycobacterium butyricum Suspension in schweren Paraffin Öl (Difco; 6 mg/kg in Paraffin Öl; Merck). Die Verbindungen 1 und 2 werden in Carboxymethylcellulose (1 % in Wasser) suspendiert und oral verabreicht. Die Verbindungen werden einmal täglich vom 1. bis zum 12. Versuchstag appliziert; dann erfolgt die Bestimmung des Pfotenvolumens und Arthritis-Index am 18. Tag.

Die Schwere der Erkrankung wird durch Messung des Pfotenvolumens beider Hinterpfoten bestimmt. Die Messung erfolgt durch die Wasserverdrängungsmethode mit einem Plethysmometer 2060 (Rhema-Labortechnik, Hofheim, Deutschland). Ferner erfolgt die Bestimmung des Arthritis Index am 18. Tag nach Injektion.

Bestimmung des Arthritis Index:
- 1. Ohren: 0,5 Punkte für jedes Ohr an dem eine Rötung auftritt und Knoten gebildet werden
- 2. Nase: 1 Punkt für Bindegewebsschwellung
- 3. Schwanz: 1 Punkt für das Auftauchen von Knoten
- 4. Vorderpfoten: 0,5 Punkte für jede Pfote an der wenigsten eine Entzündung an einem Gelenk auftritt
- 5. Hinterpfoten: 1 Punkt für leichte Entzündung (Schwellung) 2 Punkte für eine mittelstarke Entzündung 3 Punkte für eine massive Entzündungsreaktion

Tiere einer Kontrollgruppe erhalten nur das Lösungsmittel (1 % Carboxymethylcellulose in Wasser). Pro Dosierung und in der Kontrollgruppe werden jeweils 6 Tiere verwendet. Als Wirkungskriterium dient die Herabsetzung der Pfotenvolumenzunahme und die Abnahme des Arthritis Index gegenüber der unbehandelten Kontrollgruppe.

Tabelle 1 zeigt die Ergebnisse. Die Gesamtmenge der Verbindungen 1 und 2 ist bei den verschiedenen Experimenten jeweils konstant.

**Tabelle 1**

| Verbindung 1 (mg/kg Ratte) | Verbindung 2 (mg/kg Ratte) | Abnahme des Pfotenvolumens (%) | Abnahme des Arthritis Index (%) |
|---|---|---|---|
| 10 | 0 | 74 | 58 |
| 9,9 | 0,1 | 93 | 66 |
| 9,7 | 0,3 | 94 | 71 |
| 9,0 | 1,0 | 95 | 66 |
| 5 | 0 | 10 % Zunahme | 12 % Zunahme |
| 4,85 | 0,15 | 10 | 5 |
| 4,5 | 0,5 | 46 | 35 |

Durch zunehmende Mengen der Verbindung 2 wird sowohl bei 5 mg/kg Lebendgewicht der Ratte als auch bei 10 mg/kg die Wirkung der erfindungsgemäßen Zubereitung deutlich gesteigert. Daher führen geringe zusätzliche Mengen von Verbindung 2 zu einer deutlichen Wirkungssteigerung der erfindungsgemäßen Zubereitung.

## Patentansprüche

1. Feste Zubereitung, enthaltend
die Komponente 1) 5-Methylisoxazol-4-carbonsäure-(4-trifluormethyl)-anilid,
die Komponente 2) die Verbindung der Formel I 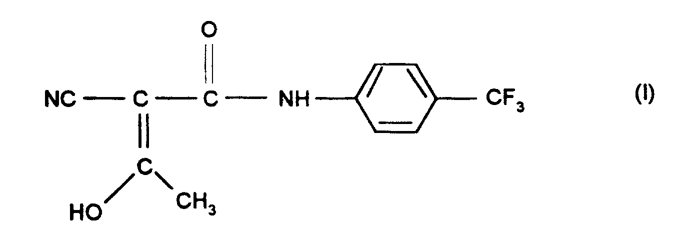 und/oder eine stereoisomere Form der Verbindung der Formel I und/oder ein physiologisch verträgliches Salz der Verbindung der Formel I, und
3) einen pharmazeutisch verträglichen Träger,
dadurch gekennzeichnet, daß der Gehalt der Komponente 1 von 2 bis 20 mg beträgt und der Gehalt der Komponente 2) von 0,3 % bis 50 % der Komponente 1) beträgt.

2. Zubereitung gemäß Anspruch 1, dadurch gekennzeichnet, daß der Gehalt der Komponente 2) von 0,5 % bis 20 % der Komponente 1) beträgt.

3. Zubereitung gemäß der Ansprüche 1 oder 2, dadurch gekennzeichnet, daß der Gehalt der Komponente 2) von 0,8 bis 15 % der Komponente 1) beträgt.

4. Zubereitung gemäß einem oder mehreren der Ansprüche 1 bis 3, dadurch gekennzeichnet, daß der Gehalt der Komponente 2) von 1 % bis 10 % der Komponente 1 beträgt.

5. Zubereitung gemäß einem oder mehreren der Ansprüche 1 bis 4, dadurch gekennzeichnet, daß der Gehalt der Komponente 2) von 1 % bis 5 % der Komponente 1 beträgt.

6. Zubereitung gemäß einem oder mehreren der Ansprüche 1 bis 5, dadurch gekennzeichnet, daß es die Komponenten 1 und 2 in einer Darreichungsform zur rektalen oder oralen Applikation enthält.

7. Zubereitung gemäß einem oder mehreren der Ansprüche 1 bis 6, dadurch gekennzeichnet, daß die Komponenten 1 und 2 in gleichartigen, getrennten Darreichungsformen zur zeitlich gemeinsamen Verabreichung vorliegen.

8. Zubereitung gemäß einem oder mehreren der Ansprüche 1 bis 7, dadurch gekennzeichnet, daß die Komponenten 1 und 2 in getrennten, unterschiedlichen Darreichungsformen zur zeitlich gemeinsamen Verabreichung vorliegen.

9. Verwendung der Zubereitung gemäß einem oder mehreren der Ansprüche 1 bis 8 zur Herstellung eines Arzneimittels zur Behandlung von immunologischen Erkrankungen.

10. Verwendung der Zubereitung gemäß einem oder mehreren der Ansprüche 1 bis 8 zur Herstellung eines Arzneimittels zur Behandlung von akuten immunologischen Ereignissen wie Sepsis, Allergie, Graft-versus-Host-Reaktionen oder Host-versus-Graft-Reaktionen oder Autoimmunerkrankungen wie rheumatoide Arthritis, systemischem Lupus erythematodes oder multipler Sklerose oder Psoriasis, atopischer Dermatitis, Asthma, Urtikaria, Rhinitis, Uveitis, Typ II-Diabetes, zystischer Fibrose, Kolitis, Leberfibrose oder Krebserkrankungen wie Lungenkrebs, Leukämie, Eierstockkrebs, Sarkome, Kaposi's Sarkom, Meningiom, Darmkrebs, Lymphknotenkrebs, Hirntumore, Brustkrebs, Pankreaskrebs, Prostatakrebs oder Hautkrebs.

11. Verfahren zur Herstellung der Zubereitung gemäß einem oder mehreren der Ansprüche 1 bis 8, dadurch gekennzeichnet, daß man 5-Methylisoxazol-4-carbonsäure-(4-trifluormethyl)-anilid, die Verbindung der Formel I und/oder ein physiologisch verträgliches Salz der Verbindung der Formel I und/oder eine stereoisomere Form der Verbindung der Formel I und einen pharmazeutischen Träger zu einer pharmazeutischen Darreichungsform verarbeitet.

## Claims

1. A solid preparation, comprising
component 1) 5-methyl-4'-trifluoromethyl-4-isoxazolecarboxanilide,
component 2) the compound of the formula I 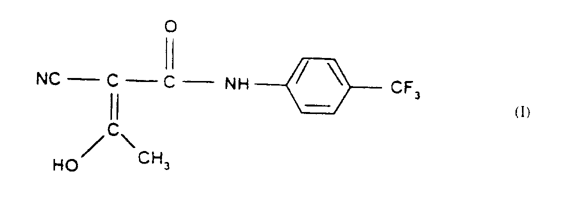 and/or a stereoisomeric form of the compound of the formula I and/or a physiologically tolerated salt of the compound of the formula I, and
3) a pharmaceutically tolerated excipient,
wherein the content of component 1) is from 2 to 20 mg and the content of component 2) is from 0.3% to 50% of that of component 1).

2. The preparation as claimed in claim 1, wherein the content of component 2) is from 0.5% to 20% of that of component 1).

3. The preparation as claimed in claims 1 or 2, wherein the content of component 2) is from 0.8 to 15% of that of component 1).

4. The preparation as claimed in one or more of claims 1 to 3, wherein the content of component 2) is from 1% to 10% of that of component 1).

5. The preparation as claimed in one or more of claims 1 to 4, wherein the content of component 2) is from 1% to 5% of that of component 1).

6. The preparation as claimed in one or more of claims 1 to 5, which comprises components 1) and 2) in an administration form for rectal or oral administration.

7. The preparation as claimed in one or more of claims 1 to 6, wherein components 1) and 2) are present in similar, separate administration forms for being administered at the same time.

8. The preparation as claimed in one or more of claims 1 to 7, wherein components 1) and 2) are present in separate, different administration forms for being administered at the same time.

9. The use of the preparation as claimed in one or more of claims 1 to 8 for preparing a pharmaceutical for treating immunological diseases.

10. The use of the preparation as claimed in one or more of claims 1 to 8 for preparing a pharmaceutical for treating acute immunological events, such as sepsis, allergy and graft-versus-host reactions or host-versus-graft reactions, or autoimmune diseases, such as rheumatoid arthritis, systemic lupus erythematosus or multiple scelerosis, or psoriasis, atopic dermatitis, asthma, urticaria, rhinitis, uveitis, type II diabetes, cystic fibrosis, colitis or hepatic fibrosis, or cancerous diseases, such as lung cancer, leukemia, ovarian cancer, sarcoma, Kaposi's sarcoma, meningioma, intestinal cancer, lymph node cancer, brain tumours, breast cancer, pancreatic cancer, prostate cancer or skin cancer.

11. A process for producing the preparation as claimed in one or more of claims 1 to 8, which comprises processing 5-methyl-4'-trifluoromethyl-4-isoxazolecarboxanilide, the compound of the formula I and/or a physiologically tolerated salt of the compound of the formula I and/or a stereoisomeric form of the compound of the formula I and a pharmaceutical excipient into a pharmaceutical administration form.

## Revendications

1. Composition solide, contenant
le composant 1) 5-méthylisoxazole-4-carboxy-(4-trifluorométhyl)anilide,
le composant 2) le composé de formule I 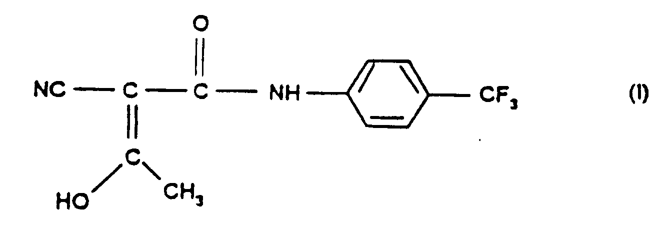 et/ou une forme stéréoisomère du composé de formule I et/ou un sel physiologiquement acceptable du composé de formule I, et
3) un véhicule pharmaceutiquement acceptable,
caractérisé en ce que la teneur en le composant 1) va de 2 à 20 mg et la teneur en le composant 2) va de 0,3 à 50 % de celle du composant 1).

2. Composition selon la revendication 1, caractérisée en ce que la teneur en le composant 2) va de 0,5 % à 20 % de celle du composant 1).

3. Composition selon la revendication 1 ou 2, caractérisée en ce que la teneur en le composant 2) va de 0,8 à 15 % de celle du composant 1).

4. Composition selon une ou plusieurs des revendications 1 à 3, caractérisée en ce que la teneur en le composant 2) va de 1 % à 10 % de celle du composant 1).

5. Composition selon une ou plusieurs des revendications 1 à 4, caractérisée en ce que la teneur en le composant 2) va de 1 % à 5 % de celle du composant 1).

6. Composition selon une ou plusieurs des revendications 1 à 5, caractérisée en ce qu'elle contient les composants 1 et 2 en une forme d'administration destinée à l'administration orale ou rectale.

7. Composition selon une ou plusieurs des revendications 1 à 6, caractérisée en ce que les composants 1 et 2 se trouvent sous des formes d'administration identiques séparées, pour l'administration conjointement dans le temps.

8. Composition selon une ou plusieurs des revendications 1 à 7, caractérisée en ce que les composants 1 et 2 se trouvent sous des formes d'administration séparées différentes, pour l'administration conjointement dans le temps.

9. Utilisation de la composition selon une ou plusieurs des revendications 1 à 8, pour la fabrication d'un médicament destiné au traitement de maladies immunitaires.

10. Utilisation de la composition selon une ou plusieurs des revendications 1 à 8, pour la fabrication d'un médicament destiné au traitement d'incidents immunologiques aigus tels que l'état septique, l'allergie, des réactions du greffon contre l'hôte ou des réactions de l'hôte contre le greffon, ou de maladies auto-immunes telles la polyarthrite rhumatoïde, le lupus érythémateux disséminé ou la sclérose en plaques ou le psoriasis, la dermatite atopique, l'asthme, l'urticaire, la rhinite, l'uvéite, le diabète de type II, la fibrose kystique, la colite, la fibrose hépatique, ou de maladies cancéreuses telles que le cancer du poumon, la leucémie, le cancer de l'ovaire, des sarcomes, le sarcome de Kaposi, un méningiome, le cancer de l'intestin, le cancer des ganglions lymphatiques, des tumeurs cérébrales, le cancer du sein, le cancer du pancréas, le cancer de la prostate ou le cancer de la peau.

11. Procédé pour la préparation de la composition selon une ou plusieurs des revendications 1 à 8, caractérisé en ce que l'on met sous une forme d'administration pharmaceutique du 5-méthylisoxazole-4-carboxy-(4-trifluorométhyl)anilide, le composé de formule I et/ou un sel physiologiquement acceptable du composé de formule I et/ou une forme stéréoisomère du composé de formule I, et un véhicule pharmaceutique.
